# EUROPEAN PATENT APPLICATION

(11) **EP 4 610 344 A1**
(43) Date of publication of application: **03.09.2025**
(21) Application number: 24160723.3
(22) Date of filing: 29.02.2024
(51) Int. Cl.: C12M 1/34, B01L 3/00, G01N 33/483

(54) **A DEVICE, SYSTEM, METHOD AND COMPUTER PROGRAM FOR PROCESSING BIOLOGICAL MATERIAL**

(71) Applicant: Imec VZW, 3001 Leuven (BE)
(72) Inventor: Mora Lopez, Carolina, 3001 Heverlee (BE)
(74) Representative: Patent Department IMEC

(57) **Abstract**

The invention relates to a device (100) for processing biological material comprising: an array (110) of pixels (10) comprising one or more rows of pixels, wherein each row of pixels comprises a first and a last pixel (10a, 10b), wherein each pixel (10) comprises a pixel circuit (11) and an electrode (12) for interacting with the biological material, wherein the pixel circuit (11) is electrically coupled to the electrode (12) and wherein each pixel circuit (11) is configured to process the biological material by receiving an input signal from the biological material via the electrode (12) and/or by providing an output signal to the biological material via the electrode (12); a data controller (101); a plurality of electrical interconnections (21 to 29) configured for bi-directional communication between neighboring pixel circuits (11) within a row, between the data controller (101) and the pixel circuit (11) of the first pixel (10) of each row, and between the data controller (101) and the pixel circuit (11) of the last pixel (10) of each row; and wherein the data controller (101) is configured to be switchable between a first and a second connection path for commanding any of the pixel circuits (11) in any of the rows to process the biological material, wherein the first connection path is via the pixel circuit (11) of the first pixel (10a) of the row and the second connection path is via the pixel circuit (11) of the last pixel (10b) of the row.

## Description

### Technical Field

The present invention relates to a device, system, method and computer program for processing biological material. In particular, the present invention relates to a device, system, method and computer program for processing biological materials in vitro on a surface of the device.

### Background

Studying biological material may be of interest in many cell-physiological and medical applications. In order to study biological material, it is typically cultured in vitro.

A device for analysis of biological material may be provided with a microelectrode array, wherein circuitry is arranged in an active sensor area, on which the biological material is placed. Thus, the circuitry of the microelectrode array may form contact with the biological material for analyzing biological material.

One known technique for processing biological material via microelectrode array is explained in Patent Application US 2020/0018742 A1.

### Summary

The invention is set out in the appended set of claims.

It is an objective to improve the circuitry in the prior art. In particular, it is an object to provide robustness to the circuitry for processing biological material.

In the first aspect, the present description relates to a device for processing biological material. The device comprises: an array of pixels comprising one or more rows of pixels, wherein each row of pixels comprises a first and a last pixel, and each pixel comprises a pixel circuit and an electrode for interacting with the biological material. The pixel circuit is electrically coupled to the electrode and wherein each pixel circuit is configured to process the biological material by receiving input signal from the biological material via the electrode and/or by providing output signal to the biological material via the electrode. The device comprises a data controller and a plurality of electrical interconnections. The electrical interconnections are configured for bi-directional communication between neighboring pixel circuits within a row, between the data controller and the pixel circuit of the first pixel of each row, and between the data controller and the pixel circuit of the last pixel of each row. The data controller is configured to be switchable between a first and a second connection path for commanding any of the pixel circuits in any of the rows to process the biological material, wherein the first connection path is via the pixel circuit of the first pixel of the row and the second connection path is via the pixel circuit of the last pixel of the row.

It is an advantage that the electrical interconnections provide redundancy in operation for communication between each pixel circuit in a pixel and the data controller. Each pixel circuit in a pixel is possible to be communicated via the first or the second connection path.

According to an example embodiment, the data controller is configured for communicating with a first set of pixel circuits of any row via the first connection path, and communicating with a second set of different pixel circuits of the row via the second connection path. It is an advantage of the invention that the two sets of pixel circuits in a row can be communicated simultaneously without any disturbance between the two sets. It is an advantage of the invention that the efficiency of biological material processing is increased. In some example embodiments, the efficiency can be doubled. According to an example embodiment, such division of the first and second sets of pixel circuits is due to a physical breakage or malfunction of an electrical interconnection. According to an example embodiment, such division can also be manually set.

According to an example embodiment, each pixel circuit further comprises: an analog-to-digital converter configured to digitize the analog input signal received from the electrode. A signal output line interconnecting the pixel circuits is adapted for serial communicating the signal digitized by the analog-to-digital converter to the data controller.

According to another example embodiment, each pixel circuit further comprises: a digital-to-analog converter configured to convert a digital output signal received from the data controller into an analog output signal and to provide the analog output signal to the electrode. A signal input line interconnecting the pixel circuits is adapted for serial communicating the digital output signal received from the data controller to the digital-to-analog converter. According to an example embodiment, each pixel circuit comprises an analog-to-digital converter or a digital-to-analog converter.

According to a yet another example embodiment, each pixel circuit comprises an analog-to-digital converter and a digital-to-analog converter.

It is an advantage that each pixel circuit converting analog signal locally. Thus, only digitized signals are communicated between the pixel circuits, and between the row of pixels and the data controller. The present invention further allows the device to have pixel array with large number of pixels. According to an example embodiment, the device comprises 100*100 pixels. According to an example embodiment, the device comprises 1000*1000 pixels.

According to an example embodiment, the electrical interconnections between the data controller and the first or last pixel circuit of any row of pixels as well as the electrical interconnections between neighboring pixel circuits in each row comprise switches for interrupting said electrical interconnections. It is an advantage of the invention that electrical interconnections can be manually switched off between the pixel circuits. Thus, the row of pixels is divided manually to have a first and second sets of pixel circuits.

According to an example embodiment, the array of pixels comprises a plurality of rows of pixels, defining columns of pixels. Each of the pixel circuits in a column of the array is individually electronically coupled to a power rail and a ground rail, wherein the power rail and the ground rail extend along the column of pixels. It is an advantage of the invention that redundancy is also provided for power connections. Each of the pixel circuits connects to the common power and ground rail. According to an example embodiment, when a power and/or ground rail between two neighboring pixel circuit is broken or malfunctioning, each of the neighboring pixel circuit can be still receive power supply from another neighboring pixel circuit.

According to an example embodiment, the device further comprises a rigid mesh structure. The structure comprises: island structures, wherein each pixel is comprised in an island structure. The structure further comprises bridge structures physically interconnecting the island structures, wherein the electrical interconnections between neighboring pixel circuits are comprised in the bridge structures. The island structures and the bridge structures define through-pores. It is an advantage of the invention that such rigid mesh structure provides possibility to monitor and/or stimulate cells inside a biological material traversing through the mesh structure. The mesh structure may be fragile. The pixel circuits provide robust communication in the fragile rigid mesh structure to ensure communication even when a bridge between islands is broken.

According to an example embodiment, the rigid mesh structure comprises a semiconductor material. It is an advantage that the device with semiconductor material can be manufactured economically with CMOS manufacture processes. It is an advantage that the device in semiconductor material is compatible with biological material. According to an example embodiment, at least one component in the pixel circuits comprises semiconductor material.

In a second aspect, the description relates to a method for processing biological material interacting with electrodes of the device in the first aspect, comprising steps of: causing the date controller to send a command signal to a pixel circuit via either the first or the second connection path; thereby causing the pixel circuit to process the biological material.

The command signal is sent in serial communication.

It is an advantage that the method requires limited number of communication lines for communication between pixel circuits and the data controller because of serial communication.

According to an example embodiment, the method further comprises the steps of: causing the data controller to deliver an interrogation signal to the first pixel of at least one of the one or more rows, causing the data controller to instruct each pixel having received an interrogation signal but the last pixel of the at least one of the one or more rows to attempt sending an interrogation signal to the next pixel in the at least one of the one or more rows and to send a reply signal to the previous pixel in the at least one of the one or more rows or to the data controller if there is no previous pixel in the at least one of the one or more rows, causing the data controller to configure itself to command the process of the biological material via the first connection path by any pixel having sent a reply signal, and to command the process of the biological material via the second connection path by any pixel having not sent a reply signal within a predetermined timeout period. It is an advantage of the invention that a calibration process can be integrated in the process by sending interrogation signals and test whether all electrical connections are functioning well in a row. According to an example embodiment, the calibration process can be a separate set of steps than the steps of processing the biological material. According to an example embodiment, the calibration process is conducted before the steps of processing the biological material. According to an example embodiment, the calibration process is conducted in between process cycles of processing the biological materials. Such a process cycle, as an example, can be processing all the pixels in a row or in the entire array. According to an example embodiment, the calibration process is conducted before and during the process of the biological materials. According to an example embodiment, the calibration process can be integrated in the steps of processing biological material. In an example embodiment, the command signal, sent from the data controller to each pixel circuit, comprises an interrogation signal. In an alternative embodiment, the command signal functions as an interrogation signal. It is an advantage that the status of the array in the device can be monitored frequently and, in some cases, even in real-time.

According to an example embodiment, the electrical interconnections between the data controller and any row of pixels as well as between neighboring pixel circuits in each row comprise switches for interrupting said electrical interconnections. The method further comprises a step before the step of causing the date controller to send command signal to a pixel circuit of: causing the data controller to interrupt the electrical interconnections between two neighboring pixels of the row of pixel, thereby forming a first set of pixels commendable by the first connection path and a second set of pixels commendable by the second connection path. It is an advantage that when all the electrical interconnections work well in a row of pixel, the pixels can be divided manually to two separate sets of pixels and the two sets of pixels can process the biological material in two different pixels in a row simultaneously.

According to an example embodiment, the step of causing the data controller to interrupt the electrical interconnections between two neighboring pixels of the row of pixel is performed so that the difference in the number of pixels in the first and second sets of pixels is not more than 1. It is an advantage that the biological material is efficiently processed.

According to an example embodiment, the command signal instructs the pixel circuit to receive an analog input signal from the biological material from the electrode, the method further comprises the steps of: receiving the analog input signal from the electrode having contact to the biological material; digitizing the analog input signal; transmitting in serial communication the digitized input signal to the data controller via the first or second connection path.

According to an example embodiment, the command signal instructs the pixel circuit to stimulate the biological material via the electrode, the method further comprises steps of: providing a digital stimulation signal for stimulating the biological material; converting the digital stimulation signal to analog stimulation signal; and providing the analog stimulation signal to the biological material via the electrode.

In a third aspect, the description relates to a computer program comprising instructions to cause any of the device in the first aspect to execute the steps of the method in the second aspect.

In a fourth aspect, the description relates to a system comprising: a plurality of devices in the first aspect comprising island structures, wherein each pixel is comprised in an island structure, and bridge structures physically interconnecting the island structures, wherein the electrical interconnections between neighboring pixel circuits are comprised in the bridge structures. The island structures and the bridge structures define through-pores. The rigid mesh structures of the devices are stacked to form a three-dimensional system for processing biological material.

It is an advantage that the system can process the biological material not only in the planar mesh structure, but also in the vertical plane traversing the planar mesh structure.

### Brief description of the drawings

Figure 1a is a schematic illustration of a first device according to an exemplary embodiment.
Figure 1b is a schematic illustration of a pixel in the first device according to an exemplary embodiment and the connection between the pixel circuits in the pixel and the power and ground rails.
Figures 2a and 2b are zoomed in schematic illustrations of a first and second exemplary pixel circuits in the first device according to the exemplary embodiment.
Figure 3 is a schematic illustration of a second device according to an exemplary embodiment where two power/ground interconnections are malfunctioning.
Figure 4 is a schematic illustration of a rigid mesh structure of a third device according to an exemplary embodiment.
Figure 5 is a schematic illustration of a fourth device according to an exemplary embodiment where two electrical interconnections between the pixels in the pixel array are malfunctioning.
Figure 6 is a schematic illustration of a fifth device according to an exemplary embodiment where the electrical interconnections between two neighboring pixels of the rows of pixels are interrupted.

### Detailed description

The disclosure will be further elucidated by means of the following description and the appended figures. Various exemplary embodiments are described herein with reference to the following figures, wherein like numeral denotes like entities. The figures described are schematic and are non-limiting. Further, any reference signs in the claims shall not be construed as limiting the scope of the present disclosure. Still further, in the different figures, the same reference signs refer to the same or analogous elements.

The term "comprising", used in the claims, should not be interpreted as being restricted to the means listed thereafter; it does not exclude other elements or steps. It is thus to be interpreted as specifying the presence of the stated features, integers, steps, or components as referred to, but does not preclude the presence or addition of one or more other features, integers, steps or components, or groups thereof. The term "comprising" therefore covers the situation where only the stated features are present (and can therefore always be replaced by "consisting of" in order to restrict the scope to said stated features) and the situation where these features and one or more other features are present. The word "comprising" according to the invention therefore also includes as one embodiment that no further components are present. Thus, the scope of the expression "a device comprising means A and B" should not be interpreted as being limited to devices consisting only of components A and B. It means that with respect to the present invention, the only relevant components of the device are A and B.

The term "a" shall be interpreted as a function word before a mass noun to denote a particular type or instance. It should not be interpreted as a function word before a singular noun referring to one object.

Biological material includes but is not limited to biological organisms, biological tissues, microorganisms, cells, cell lines.

Analysis of electrophysiology of biological material may be of interest in many different applications. The study of electrophysiology may be particularly useful in preclinical drug discovery in order to determine how certain biological material would react when treated by a drug to be tested.

In order to improve the speed of analysis of biological material, multielectrode arrays (MEAs) have been introduced, which may provide a high throughput, especially for in vitro measurements of extracellular (ExC) action potentials (APs).

It is also of interest to enable detection of intracellular (InC) APs. Access to InC APs within biological material comprising cells for reliable detection of InC voltage may be achieved by highly localized electroporation. The electroporation of cells may thus allow low-impedance electrical recording of the InC voltage.

In addition, impedance measurements may also be used to study biological materials, such as for the study of cell morphology, adhesion, differentiation, and contractility in the biological material. Impedance measurement may include impedance spectroscopy, wherein the impedance of a cell is determined for a plurality of frequencies in a range of frequencies. ExC/InC recording, and impedance measurements may also be complementary for determining drug toxicity.

Furthermore, electric stimulation may also be used to study and guide the behavior of cells in biological material in in vitro systems based on the response of these cells to an electric field.

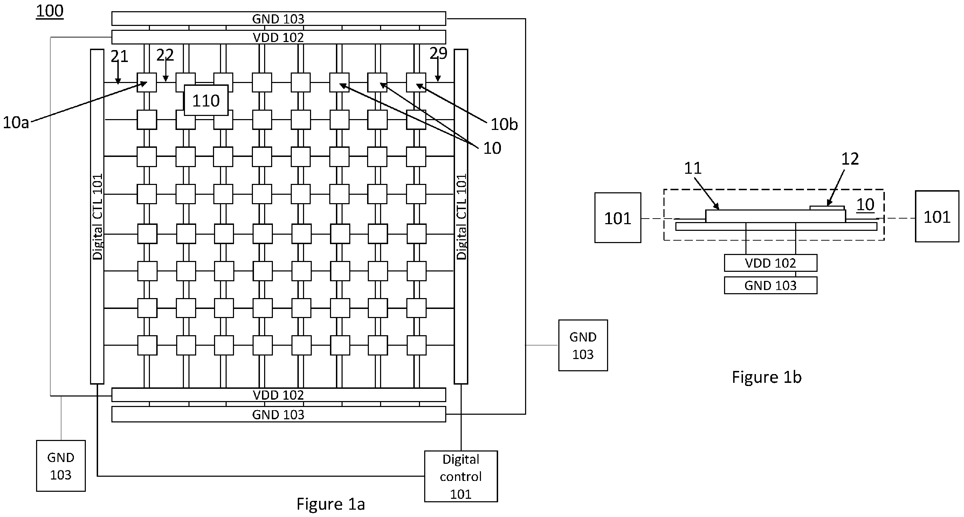

As shown in figure 1a, in an embodiment of the first aspect of the invention, a device 100 comprises a pixel array 110.

As used herein, the term "pixel" should be construed as an addressable element, which may be used for detecting an electrical signal from or providing a stimulation signal to the biological material. The plurality of pixels may thus form information, for example in two dimensions, corresponding to the biological material associated with each spatial position of a pixel in the array. The information in two dimensions is thus based on the signals acquired from individual pixels and could be used to, e.g., form "an image" as a representation of detected or determined characteristics in the two spatial dimensions. However, the pixel should not be construed as necessarily determining an intensity of light, but rather the pixels determine electrical signals relating to cells, which may be converted to information about the cells.

The pixel array 110 comprises at least one row of pixels and a plurality of columns of pixels 10. According to an example embodiment, the pixel array 110 contains only one row of pixels. According to another example embodiment, the pixel array 110 is a two-dimensional array comprising a plurality of rows of pixels 10. The terms "row" and "column" refer to substantially straight lines of pixels 10. A column refers to a line of pixels 10 vertically from top to bottom. A row refers to a line of pixels 10 horizontally from left to right. The column and the row intersect at the pixel 10 in the pixel array 110. The pixels 10 in the row have electronic connections between the pixel circuits 11 of the neighboring pixels 10. the pixels 10 in the column can have electronic connections between the pixel circuits 11 of the neighboring pixels 10. The interpretation of the term "row" and "column" is interchangeable. In an example embodiment, the rows and columns can be substantively perpendicular. A row of pixels 10 has two end pixels 10a, 10b. One of the two end pixels 10 is defined as the first pixel 10a and naturally the other pixel 10 is defined as the last pixel 10b. Because the nature of the pixels is the same, the interpretation of the term "first pixel" and "last pixel" is interchangeable.

Each pixel 10, as shown in figure 1b, comprises a pixel circuit 11 and an electrode 12 for interacting with biological material. According to an example embodiment, the electrode 12 has direct contact with the biological material. The electrode 12 is biologically compatible for having direct contact with the biological material. The pixel circuit 11 is electrically coupled to the electrode 12. According to an example embodiment, the pixel circuit 11 is not exposed to the biological material thus forms no direct contact with the biological material. According to an example embodiment, the pixel circuit 11 is configured to process data locally in each pixel 10.

Device 100 further comprises a data controller 101 for instructing the pixel circuits 11 in the pixels 10. The pixel circuits 11 are connected by electrical interconnections 21 to 29 between the neighboring pixel circuits 11 in a row. The pixel circuits 11 of the first 10a and last pixels 10b are connected via electrical interconnections to the data controller 101. According to an example embodiment, the electronic interconnection comprises communication lines, such as metal lines or metal wires. Such a communication line can deliver signals bi-directionally between the pixel circuits 11 in the neighboring pixels or between the pixel circuit 11 of the first/last pixel 10a, 10b and the data controller 101. As a consequence, each pixel circuit 11 has and only has two bi-directional communication connections to the two neighboring pixel circuits 11 in a row: one to a first neighboring pixel circuit 11 and the other to another neighboring pixel circuit 11 or, one to one pixel circuit 11 of the neighboring pixel 10 and one to the data controller 101. The pixel circuits 11 will receive the instructions from the data controller 101 via one of the bi-directional communication connections. Thus, the data controller 101 can instruct a pixel circuit 11 in the row of pixel 10 via one of the pixel circuits 11 of the neighboring pixels 10 in a first connection path via the pixel circuit 11 in the first pixel 10a or in a second connection path via the pixel circuit 11 in the last pixel 10b. The first connection path refers to all the pixels and the electronic interconnections in one side of the pixel 10 in the row including the first pixel 10a. The second connection path refers to all the pixels and the electronic interconnections in the other side of the pixel 10 in a row including the last pixel 10b. The data controller 101 can communicate directly with the pixel circuit in the first pixel 10a. The data controller 101 can communicate to the pixel circuit of the next subsequent neighboring pixel via the first pixel 10a. The communication signal from the data controller 101 is first delivered to the pixel circuit in the first pixel 10a and the first pixel 10a will deliver the communication signal to the subsequent neighboring pixel. Thus, as an example, the data controller 101 can even communicate to the pixel circuit of the last pixel 10b via the first pixel 10a and all the pixels between the first 10a and last pixel 10b. The data controller 101 can directly communicate to the pixel circuit of the last pixel from the other side. Here "direct" communication between the pixel circuit of a pixel and the data controller 101 refers to a communication which does not involve another pixel in-between. And "indirect" communication between the pixel circuit of a pixel and the data controller 101 refers to a communication which must involve another pixel in-between. The data controller 101 is configured to be switchable between the first and the second connection path for commanding any of the pixel circuits 11 in any of the rows to process the biological material.

The switchable connection paths advantageously provide redundancy in the communication between the pixels 10 and the data controller 101. Even if a pixel is not reachable via one of the connection paths, it might be still reached via the other connection path.

According to an example embodiment, the pixel circuit 11 is configured to record input signals via the corresponding electrode 12 sensing the biological material. According to an example embodiment, the pixel circuit 11 is configured to provide stimulation output signals via the corresponding electrode 12 processing the biological material. According to an example embodiment, the pixel circuit 11 is configured to record input signals via the corresponding electrode 12 sensing the biological material and provide stimulation output signals via the corresponding electrode 12 processing the biological material. For example, the pixel circuit 11 is configured to alternately provide output signals and record input signals. According to an example embodiment, the pixel circuit 11 is electrically coupled to more than one electrode 12 for providing output signals and/or recording input signals.

According to an example embodiment, the data controller 101 is configured for communicating with a first set of pixel circuits of a row via the first connection path and communicating with a second set of different pixel circuits of the same row via the second connection path. The first set of pixel circuits comprises the pixel circuit of the first pixel 10a. And the second set of pixel circuits comprises the pixel circuit of the last pixel 10b. The first and second sets of pixel circuits do not have overlapping pixel circuits. The data controller 101 communicates all pixel circuits in the first set via the pixel circuit of the first pixel 10a. And the data controller 101 communicates all pixel circuits in the second set via the pixel circuit of the first pixel 10a. The first and second sets of pixel circuits can communicate with data controller 101 in a completely separated manner. In an example, the communication or processing of the first set of pixels can operated at the same time as the communication or processing of the second set of pixels.

According to an example embodiment, the data controller 101 is configured for communicating with all pixel circuits 11 of a row via the first connection path and communicating with all the pixel circuits 11 of a different row via the second connection path.

The electrical interconnections provide a plurality of communication lines for bi-directional communications between neighboring pixel circuits 11. The communication lines can be control lines between local digital control units, or common clock lines and input/output signal lines.

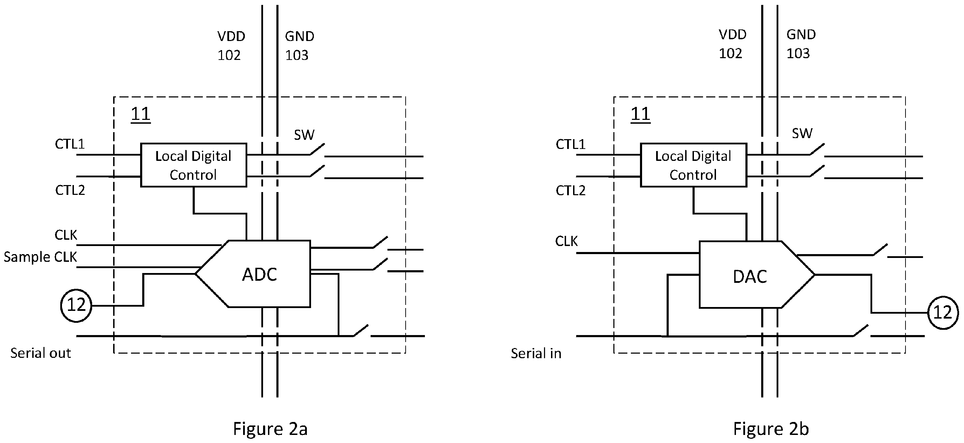

According to an example embodiment, as shown in figure 2a and 2b, the pixel circuit 11 comprise digital processing components. According to an example embodiment, the pixel circuit 11 comprises a local digital control unit configured to receive and/or pass along the command signals from the data controller 101. The digital control unit is connected to the neighboring pixels 10 and/or data controller 101 via at least one control communication line. The digital control unit is connected to the neighboring pixels 10 and/or data controller 101 via two control lines. The control lines can be used for resetting the pixel circuit 11, scanning and assessing the pixel circuit status and functionality. The data controller 101 is electrically coupled to other components in the pixel circuit 11 and is configured to instruct the components in digital signals.

According to an example embodiment, as shown in figure 2a, the pixel circuit 11 comprises an analog-to-digital converter (ADC). The ADC is electrically coupled to the electrode and receives the analog signal recorded therefrom. The pixel circuit 11 communicates the processed signal from the ADC to the data controller 101 in serial communication via the serial output line as one of the bi-directional communication lines. The ADC requires a further global clock signal from the data controller 101 in a clock communication line CLK and a sample signal in a sample communication line (Sample CLK). The clock signal and the sample signal are communicated in serial communication. Serial communication refers to the process of sending data one bit at a time, sequentially, over a communication line. According to an example embodiment, there is a clock signal per pixel row. The clock frequency depends on the resolution of the ADC. According to an example embodiment, the clock frequency is around 100MHz in a 100*100 array.

Serial communication in the line further reduces the number of wires needed between the pixels in a row in the array and the data controller 101. Conventional MEA requires at least 100 metal wires per row to connect an array of 100*100 pixels 110 to the data controller 101.

According to an example embodiment, the pixel circuit 11 further comprises a local digital signal processing unit for processing the recorded signal.

According to an example embodiment, all the components in the pixel circuit beside the ADC are digital components. The pixel size can be advantageously scaled to 50*50 µm.

According to an example embodiment, the pixel circuits 11 receive only digital signals from the data controller 101 and transmit only digital signals to the data controller 101.

According to an example embodiment, as shown in figure 2b, the pixel circuit 11 comprises a digital-to-analog converter (DAC) for converting a digital output signal received from the data controller 101 to an analog stimulation signal and provide the stimulation signal to the biological material via the electrode 12. The DAC requires a further global clock signal from the data controller 102 in a clock communication line CTL1, CTL2 and a stimulation signal from a signal input line (Serial in). The clock signal and the sample signal are communicated in serial communication.

According to an example embodiment, each of the pixel circuits 11 comprises either an ADC and/or a DAC. According to another example embodiment, the device 100 comprises a plurality of pixel circuits 11, some of which comprise an ADC and/or DAC, and some do not.

According to an example embodiment, the electrical interconnections between the data controller 101 and any row of pixels 10 as well as between neighboring pixel circuits 11 in each row comprise switches SW for interrupting said electrical interconnections. Each of the communication lines in the electrical interconnections comprises a switch SW. The data controller 101 configures the switches SW such that the entire electrical interconnections between the data controller 101 and any row of pixels 10 as well as between neighboring pixel circuits 11 in each row can be completely switched off and interrupted.

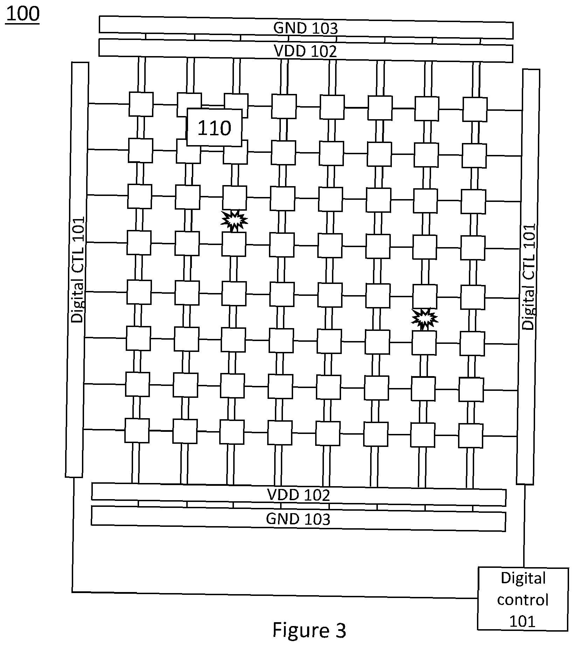

According to an example embodiment, as shown in figure 3, a two-dimensional array of pixels 110 comprises rows and columns of pixels 10. The device 100 further provides a power rail VDD 102 and a ground rail GND 103 for each column of the array 110. The power rail VDD 102 and the ground rail GND 103 extend along the column of pixels 10. Each of the pixel circuits 11 in a column of the array is individually electronically coupled to a power rail VDD 102 and a ground rail GND 103. Thus, the power rail VDD 102 and the ground rail GND 103 also interconnect the neighboring pixels 10 in the column respectively. According to an example embodiment, each pixel may comprise more than one connection to power/ground rails. In operation, if power or ground interconnection between two neighboring pixels in a column is malfunctioning, the two pixels can still get power or ground connection from their other neighboring pixels in the column. Because of the redundancy provided by the power/ground interconnection, all pixels 10 in a column can still remain powered even if there is one malfunctioning interconnection.

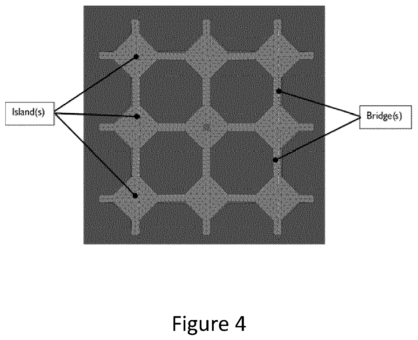

According to an example embodiment, the device 100 comprises a rigid mesh structure, as shown in figure 4, having island structures being interconnected by bridge structures and defining through-pores between the island structures allowing for selective transport of biological materials fitting through the pores such as cell constructs (e.g., cells or tissues), cellular components, proteins or other large molecules through the pores.

Thanks to the through-pores being provided by the mesh structure, a through flow can be provided through the mesh structure. This through flow may allow the formation of cell constructs that traverse the the mesh structure and extend across opposite sides of the cell culture. Hence, the island structures and bridge structures may be formed within a cell culture and extend into the interior of a three-dimensional cell culture.

Each pixel 10 in the device 100 is comprised in an island structure and the electrode 12 is exposed to have contact with the biological material. The electrical interconnections between the neighboring pixel circuits 11 in the row are comprised in the horizontal bridge structures. This may be used for analysis of interior portions of the three-dimensional cell culture.

According to an example embodiment, the power and ground interconnections between the neighboring pixel circuits 11 in the column are comprised in the vertical bridge structures.

According to an example embodiment, the rigid mesh structure comprises semiconductor material. According to an example embodiment, the rigid mesh structure comprises a silicon layer.

According to an example embodiment, the mesh structure may have a thickness below 10µm, e.g., from 1 µm to 8 µm. The thin structure makes the bridge structurally fragile, and the bridge has a high risk of sustaining physical breakage. The present invention advantageously provides redundancy and robustness in electrical connections for data transmission and/or power/ground supply. Thus, even if a bridge structure is broken, the device can still maintain the functionality of the pixels. The present invention also advantageously provides scalability because the number of metal wires is largely reduced because the data is transmitted through the pixels 10 instead of individual connection from each pixel 10 to the data controller 101.

In a second aspect, the description relates to a method for processing biological material interacting with the electrodes 12 in the device as described in the first aspect.

According to an example embodiment, the data controller 101 sends a command signal to a pixel circuit 11 via either the first or the second connection path. The pixel circuit 11 processes the biological material. The command signal is sent in serial communication.

According to an example embodiment, such steps are repeated for a plurality of pixel circuits 11.

According to an example embodiment, the data controller 101 can instruct an entire row of pixels 10 to process the biological material exclusively via the first or exclusively via the second connection path. According to an example embodiment, the data controller 101 can instruct a row of pixels to process the biological material partly via the first connection path and partly via the second connection path. For instance, the data controller 101 can instruct a first half of the pixels of a row of pixels to process the biological material via the first connection path and the other half of the pixels of the row of pixels to process the biological material via the second connection path.

According to an example embodiment, the command signal instructs the pixel circuit to receive an input signal from the biological material from the electrode 12. The method further comprises the steps of: receiving the analog input signal from the electrode 12 having contact to the biological material. Digitizing the analog input signal. Transmitting in serial communication the digitized input signal to the data controller 101 via the first or second connection path.

According to an example embodiment, the pixel circuit 11 comprises an ADC and a digital control unit. The ADC is electrically coupled to the electrode 12 and receives the analog signal recorded therefrom.

The data controller sends digital command signal to the digital control unit of the pixel 10. The digital control unit instructs the ADC to record the electrical signal via the electrode 12 and further instructs the ADC to send the processed electrical signal as data stream to the data controller 101 in serial communication in a serial output line.

According to an example embodiment, the command signal instructs the pixel circuit 11 to stimulate the biological material via the electrode 12. The method further comprises steps of: providing a digital stimulation signal for stimulating the biological material; converting the digital stimulation signal to analog stimulation signal; and providing the analog stimulation signal to the biological material via the electrode 12.

According to an example embodiment, the pixel circuit 11 comprises a DAC for converting a digital output signal received from the data controller 101 to an analog stimulation signal and providing the stimulation signal to the biological material via the electrode 12, and a digital control unit. The DAC is electrically coupled to the electrode 12. The digital control unit instructs the DAC to provide analog signals thereto.

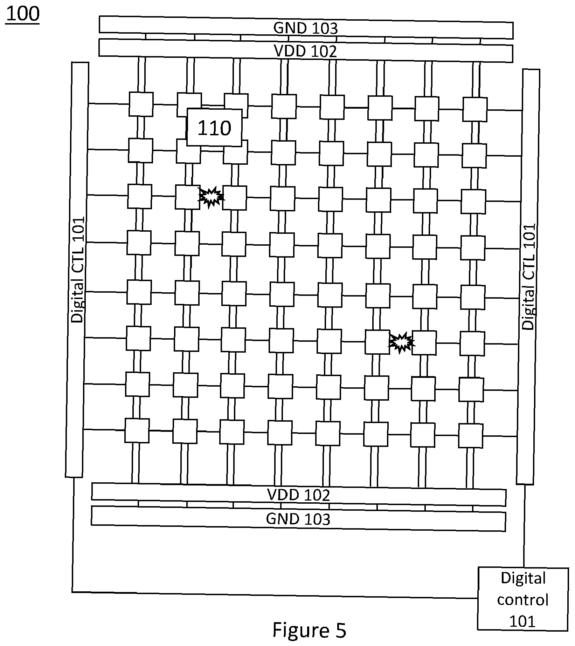

According to an example embodiment, the method further comprises an interrogation process. According to an embodiment: the data controller 101 delivers an interrogation signal to the first pixel 10 of at least one of the one or more rows. The data controller 101 instructs each pixel 10 having received an interrogation signal but the last pixel 10 of the at least one of the one or more rows to attempt sending an interrogation signal to the next pixel 10 in the at least one of the one or more rows and to send a reply signal to the previous pixel 10 in the at least one of the one or more rows or to the data controller if there is no previous pixel in the at least one of the one or more rows, causing the data controller 101, e.g., as shown in figure 5, to configure itself to command the process of the biological material via the first connection path by any pixel having sent a reply signal, and to command the process of the biological material via the second connection path by any pixel 10 having not sent a reply signal within a predetermined timeout period.

According to an example embodiment, the interrogation process can be applied as a separate calibration process to the device 100 before any steps for processing biological material.

According to an example embodiment, the interrogation process can be applied during the operation of the biological material processing. According to an example embodiment, such interrogation process is applied between sessions of the biological material processing. The sessions can be defined as a time period for biological material processing. After a predetermined time session, the data controller 101 will run the interrogation process before continuing further processing of the biological material. The session can be alternatively defined as a process cycle for biological material processing. For example, the data controller 101 will run the interrogation process after instructing all the pixels 10 in the device to process the biological material as one cycle and before instructing all the pixels 10 in the device to process the biological material as the next cycle.

According to an example embodiment, the interrogation process can be also embedded in the steps for processing biological material. In such a case, the control signal itself can be the interrogation signal and the next pixel is commanded to send back a reply signal via the first connection path. If the next pixel does not send back a reply signal within the predetermined timeout period, the pixel will send back an additional reply signal to the data controller. The data controller 101 will thus command the process of the next pixel via the second connection path.

According to an example embodiment, one of the causes of the non-reply after the predetermined timeout period is that the electrical interconnection is malfunctioning. According to an example embodiment, such malfunctioning can be the consequence of a physical breakage of the electrical interconnections such as breakage of the bridge structure in the example of the rigid mesh structure.

According to an example embodiment, the data controller 101 interrupts the electrical interconnections between two neighboring pixels 10 of the row of pixel before the step of causing the data controller 101 to send a command signal to a pixel circuit 11, thereby defining a first set of pixels commendable by the first connection path and a second set of pixels commendable by the second connection path in a row of pixels.

According to an example embodiment, the method comprises a step of determining an interruption point between two neighboring pixels of the row of pixel before the step of causing the data controller 101 to interrupt the electrical interconnections between two neighboring pixels of the row of pixel. Such interruption point is not necessarily caused by physical breakage of the electrical interconnections.

According to an example embodiment, the step of causing the data controller to interrupt the electrical interconnections between two neighboring pixels of the row of pixel is performed so that the difference in the number of pixels in the first and second sets of pixels is not more than 1.

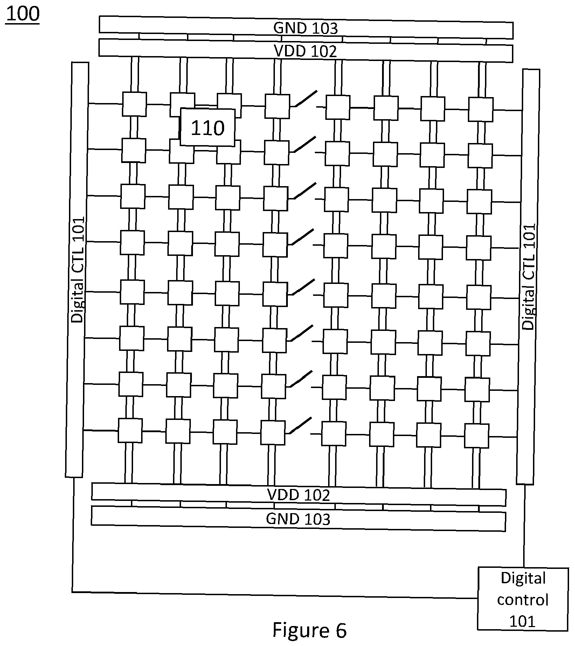

As shown in figure 6, rows of 8 pixels are evenly interrupted to two sets of 4 pixels. The first set of pixels are commendable by the data controller 101 via the first connection path. The second set of pixels are commendable by the data controller 101 via the second connection path.

In a third aspect, the description relates to a computer program configured to conduct the method as described above.

In a fourth aspect, the description relates to a system comprising a plurality of devices 100 for processing biological material according to embodiments herein described.

According to an example embodiment, each device 100 comprises a rigid mesh structure. The rigid mesh structure comprises: a. island structures, wherein each pixel 10 is comprised in an island structure, and b. bridge structures physically interconnecting the island structures, wherein the electrical interconnections between neighboring pixel circuits 11 are comprised in the bridge structures. The island structures and the bridge structures define through-pores. The rigid mesh structures of the devices 100 are stacked to form a three-dimensional system for processing biological material. According to an example embodiment, the biological material traverse the stacked mesh structure and extend through the three dimensional system.

According to an example embodiment, a common data controller 101 can be shared by the devices 100 and used as the data controller 101 for each device 100. Alternatively, a common data controller 101 can be electrically coupled to individual data controllers 101 for each device 100.

## Claims

1. A device (100) for processing biological material comprising:
- an array (110) of pixels (10) comprising one or more rows of pixels,
wherein each row of pixels comprises a first and a last pixel (10a, 10b), wherein each pixel (10) comprises a pixel circuit (11) and an electrode (12) for interacting with the biological material,
wherein the pixel circuit (11) is electrically coupled to the electrode (12) and wherein each pixel circuit (11) is configured to process the biological material by receiving an input signal from the biological material via the electrode (12) and/or by providing an output signal to the biological material via the electrode (12);
- a data controller (101);
- a plurality of electrical interconnections (21 to 29) configured for bi-directional communication between neighboring pixel circuits (11) within a row, between the data controller (101) and the pixel circuit (11) of the first pixel (10) of each row, and between the data controller (101) and the pixel circuit (11) of the last pixel (10) of each row; and
- wherein the data controller (101) is configured to be switchable between a first and a second connection path for commanding any of the pixel circuits (11) in any of the rows to process the biological material, wherein the first connection path is via the pixel circuit (11) of the first pixel (10a) of the row and the second connection path is via the pixel circuit (11) of the last pixel (10b) of the row.

2. The device (100) according to claim 1, wherein the data controller (101) is configured for communicating with a first set of pixel circuits (11) of any row via the first connection path, and communicating with a second set of different pixel circuits (11) of the row via the second connection path.

3. The device (100) according to any of the preceding claims, wherein each pixel circuit (11) further comprises:
a. an analog-to-digital converter (ADC) configured to digitize the analog input signal received from the electrode (12), and a signal output line adapted for serial communicating the signal digitized by the analog-to-digital converter (ADC) to the data controller, and/or
b. a digital-to-analog converter (DAC) configured to convert a digital output signal received from the data controller (101) into an analog output signal and to provide the analog output signal to the electrode (12), and a signal input line adapted for serial communicating the digital output signal received from the data controller (101) to the digital-to-analog converter (DAC).

4. The device (100) according to any of the preceding claims, wherein the electrical interconnections (21, 29) between the data controller (101) and the first or last pixel circuit of any row of pixels (10) as well as the electrical interconnections (22) between neighboring pixel circuits (11) in each row comprise switches (SW) for interrupting said electrical interconnections.

5. The device (100) according to any of the preceding claims,
wherein the array (110) of pixels comprises a plurality of rows of pixels (10), defining columns of pixels (10),
wherein each of the pixel circuits (11) in a column of the array is individually electronically coupled to a power rail (102) and a ground rail (103), wherein the power rail (102) and the ground rail (103) extend along the column of pixels.

6. The device (100) according to any one of the preceding claims further comprising a rigid mesh structure which comprises:
a. island structures, wherein each pixel (10) is comprised in an island structure, and
b. bridge structures physically interconnecting the island structures, wherein the electrical interconnections between neighboring pixel circuits (11) are comprised in the bridge structures,
wherein the island structures and the bridge structures define through-pores.

7. The device (100) according to claim 6, wherein the rigid mesh structure comprises a semiconductor material.

8. A method for processing biological material interacting with electrodes (12) of the device (100) according to claim 1, comprising steps of:
sending by the data controller (101) a command signal in serial communication to a pixel circuit (11) via either the first or the second connection path; thereby processing the biological material by the pixel circuit (11).

9. A method according to claim 8, the method further comprising the steps of:
the data controller (101) delivering an interrogation signal to the first pixel (10a) of at least one of the one or more rows,
the data controller (101) instructing each pixel (10) having received an interrogation signal but the last pixel (10b) of the at least one of the one or more rows to attempt sending an interrogation signal to the next pixel (10) in the at least one of the one or
more rows and to send a reply signal to the previous pixel (10) in the at least one of the one or more rows or to the data controller (101) if there is no previous pixel (10) in the at least one of the one or more rows,
the data controller (101) commanding the process of the biological material via the first connection path by any pixel (10) having sent a reply signal, and to command the process of the biological material via the second connection path by any pixel (10) having not sent a reply signal within a predetermined timeout period.

10. The method according to claim 8 for processing biological material interacting with electrodes of the device (100) according to claim 4, comprising a step before the step of the data controller (101) sending the command signal to the pixel circuit of:
the data controller (101) interrupting the electrical interconnections between two neighboring pixels (10) of the row of pixel,
thereby forming a first set of pixels (10) commandable by the first connection path and a second set of pixels (10) commandable by the second connection path.

11. The method according to claim 10, wherein the step of causing the data controller (101) to interrupt the electrical interconnections between two neighboring pixels (10) of the row of pixel (10) is performed so that the difference in the number of pixels in the first and second sets of pixels (10) is not more than 1.

12. The method according to any of the claims 8 to 11 wherein the command signal instructs the pixel circuit (11) to receive an analog input signal from the biological material from the electrode (12), the method further comprises the steps of:
receiving the analog input signal from the electrode (12) having contact to the biological material;
digitizing the analog input signal;
transmitting in serial communication the digitized input signal to the data controller (101) via the first or second connection path.

13. The method according to any of the claims 8 to 12 wherein the command signal instructs the pixel circuit (11) to stimulate the biological material via the electrode (12), the method further comprises steps of:
providing a digital stimulation signal for stimulating the biological material;
converting the digital stimulation signal to analog stimulation signal; and
providing the analog stimulation signal to the biological material via the electrode (12).

14. A computer program comprising instructions which, when the program is executed by any of the device (100) according to claims 1 to 7, cause the device (100) to carry out the method according to any of the claims 9 to 13.

15. A system comprising a plurality of devices (100) according to any of the claims 1 to 7.
